Europäisches Patentamt

(19) European Patent Office

Office européen des brevets

(11) Publication number: **0 134 845**

**A1**

## (12) EUROPEAN PATENT APPLICATION

(21) Application number: **83201354.4**

(22) Date of filing: **21.09.83**

(51) Int. Cl.⁴: **A 61 B 5/04**
**A 61 M 25/00**

(43) Date of publication of application:
**27.03.85 Bulletin 85/13**

(84) Designated Contracting States:
**DE FR GB IT**

(71) Applicant: **Spaa, Walter**
**Trompstraat 71**
**NL-2518 BM The Hague(NL)**

(72) Inventor: **Spaa, Walter**
**Trompstraat 71**
**NL-2518 BM The Hague(NL)**

(74) Representative: **Urbanus, Henricus Maria, Ir.**
**c/o Vereenigde Octrooibureaux Nieuwe Parklaan 107**
**NL-2587 BP 's-Gravenhage(NL)**

(54) **Safety catheter.**

(57) A catheter designed for causing electrical current to flow from a conductive liquid contained in the catheter through a substantial portion of the catheter wall to a conductive environment in which the catheter has been disposed. At least a substantial portion of the catheter wall is made of an electrically conductive material providing an electrical resistance through which material electrical current can be discharged to the conductive environment.

EP 0 134 845 A1

Title: Safety catheter

The invention relates to a catheter for use in invasive blood vessel examination and/or hemodynamic monitoring of patients.

In invasive examination and monitoring methods use is made of tubes called catheters.

In general, such catheters are made of an electro-insulating material and filled with an electrolytic liquid.

If the catheter is to be used for measuring the blood pressure in one of the chambers of the heart during a so-called in-heart measurement, special precautions have to be taken in order to prevent the danger of current flow through the heart muscle.

An electrical current flowing through the heart muscle may occasionally cause heart-flutter, a condition in which the heart muscle performs uncontrolled contractions (fibrillation).

During such fibrillation, there is an arrest of the pumping function of the heart which, if no appropriate counter measures are taken, will after some time have disastrous consequences for the patient and eventually cause death.

Electrical currents through the heart muscle may be caused by the fact that the liquid column in the catheter is electrically conducting to a greater or lesser extent and an (unforeseen) electrical voltage is applied from an external source to this liquid column relative to the rest of the body.

To minimize this danger, high demands particularly as to electrical reliability are imposed on electrical measuring and/or monitoring equipment to be connected to catheters having their distal end possibly located in one of the heart chambers.

However, in spite of optimally strict precautions taken, situations may occur in which, for example due to failures in the measuring equipment or the system to which it is connected, the liquid column will conduct an electrical voltage relative to the body of the patient.

Depending upon the electrical resistance of the liquid column in the catheter and the electrical voltage relative to the patient, the electrical current through this liquid column and hence through the heart muscle may exceed the maximum value that is still considered safe.

In actual practice, it is assumed that electrical currents through the heart muscle of 10 micro-amps or less may be considered safe.

However, in general a far higher maximum value is considered safe for electrical currents passing through the human thorax, i.e. the upper part of the trunk housing, inter alia, the heart and the lungs.

This maximum value is, for example, 20 milli-amps for alternating currents having frequencies exceeding 1000 cycles per second.

The reason for this large difference between the respective maximum current values (a factor 2000) should be found in the fact that, for determining the danger limit, the current density per unit of area of the heart muscle has to be taken into account.

Should the monitoring and/or measuring equipment operate properly indeed but, by coincidence, the patient himself would be contacted with an electrical voltage relative to the monitoring and/or measuring equipment, again a current may pass through the liquid column in the catheter (and hence through the heart muscle), possibly resulting in the disastrous consequences mentioned above.

By means of the invention such a dangerous situation is prevented from occurring as the electrical current through the body is deflected and reduced to a lower value before it can reach a dangerous value in the heart chambers.

The invention relates to a modification in the structure and/or material of catheters to be used for this purpose. In this manner the electrical current passing through a portion of the liquid column is prevented from passing also through the heart muscle.

By making catheters of a material having a low resistance to electrical current, it is achieved that a major portion of the current possibly passing through the liquid column will be deflected to the body, thus highly reducing the current that might pass through the heart muscle.

In such catheters, if properly constructed, the liquid column will coact with the sheath of the catheter and the medium surrounding it to form an electrical voltage divider, resulting in the current through the heart muscle being reduced to substantially negligibly low values.

In its simplest embodiment, such a catheter may be made of a material that has been made transmissive to electrical current in a presently known manner, while for special uses this material should preferably be a poor heat conductor, so that such a catheter need not deviate from existing models in so far as shape and/or structure are concerned.

In a particular embodiment, the beginning and/or end of such a catheter are made of electrically non-conductive material while the intermediate portion, which during use may be assumed to be surrounded by a blood vessel or a substance located fully or partly in one of the limbs or at some other less dangerous place in the body, is made of electrically conductive material, so that an optimally large portion of the electrical current will be discharged through the respective part of the body, instead of through the heart muscle.

In a slightly modified embodiment, the entire catheter wall is made of electrically conductive material while one or both of the ends of the catheter are surrounded by an electrically insulating material in order to prevent the occurrence of electrical contact when inadvertently touching the inserted catheter.

In a further particular embodiment, steps are taken to optimally limit current conduction through the wall of the catheter in longitudinal direction thereof without substantially reducing the electrical conductivity in transverse direction of the catheter wall.

Such an effect may be achieved by providing islands of electrically conductive material in the wall of the catheter or by making the catheter wall of a material having alternating, electrically conductive and electrically insulating portions, the electrically conductive islands or the electrically conductive portions of the catheter wall providing electrical contact between the interior of the catheter and the exterior thereof.

In a modified embodiment of such a catheter, a helical, electrically conductive strip is provided in the catheter wall, thus lengthening the overall effective length of this electrical conductor, while the electrical resistance between the beginning and the end of the catheter is increased, thereby achieving a more favourable ratio in length/thickness conduction for the electrical current.

In a further, slightly modified embodiment, this helical strip providing electrical contact between the contents of the catheter and the surroundings thereof need not extend the full length of the catheter but may be omitted at, for example, one or both of the ends of the catheter so as to reduce the risk of the end protruding from the body coming into electrical contact with an external source and to have the opposite end located in or near the heart form a higher resistance to electrical current.

In another, again slightly modified embodiment, one or more strips are provided in longitudinal direction of the catheter, which strips are electrically conductive in a direction from the inside to the outside of the catheter.

Such electrically conductive strips provided in longitudinal direction and serving to establish electrical contact between the contents of the catheter and the surroundings thereof, need not be present throughout the entire length of the catheter.

It is further possible to provide one or more electrical conductors for establishing electrical contact between the contents of the catheter and the surroundings thereof in the form of electrically conductive material woven or otherwise incorporated in the wall of the catheter so that the conductor alternately contacts the liquid within the catheter and the medium surrounding the catheter.

Such conductors need not be present throughout the entire length of the catheter.

**0134845**

A combination of two or more of the aforesaid embodiments is possible, thereby increasing the electrical resistance of the catheter wall in its longitudinal direction without correspondingly increasing the electrical resistance between the contents of the catheter and the medium surrounding this catheter.

If necessary, materials may be provided in or at the catheter wall to compensate for mechanical deterioration, if any, of the catheter according to the invention relative to conventional catheters as resulting from the modifications made thereto.

Furthermore, certain catheter constructions and/or applications may require the heat conduction through the wall to be optimally low.

To meet this requirement, a material may be used having high electrical conductivity but poor heat conductivity.

Also, the structure of the conducting members may be made so that the transport of heat is impeded by lengthening the transport path between the inner and outer surfaces of the catheter wall.

Of course, it is possible to provided conductors for the transport of electrical signals in the longitudinal direction of the catheter, which conductors may, if necessary, be electrically separated from the other conductors provided in the catheter wall.

Self-evidently, the catheters according to the invention may be rendered radiopaque.

Finally, the invention is not limited to the embodiments described above.

**0134845**

## C L A I M S
===========

1.      A catheter, characterized in that the wall of the catheter is made of electrically conductive material in such a manner that an electrical voltage inadvertently present at the liquid column in the catheter is discharged to the surroundings being in electrical contact with the outer surface of the catheter wall.

2.      A catheter according to claim 1, characterized in that a portion of the catheter is made of electrically conductive material, preferably that portion which is located in the body but not in the heart so as to prevent the catheter wall proper from functioning as a conductor for the transport of electrical current to the heart.

3.      A catheter according to one or more of the preceding claims, characterized in that one or both of the ends of the catheter are provided with an electrically insulating outer sleeve or layer.

4.      A catheter according to one or more of the preceding claims, characterized in that the wall of the catheter includes electrical conductors which are electrically separated from each other in such a manner that electrical contact is established between the contents of the catheter and the surroundings thereof, and electrical conduction of the catheter in its longitudinal direction is optimally reduced.

5.      A catheter according to one or more of the preceding claims, characterized in that the electrical conductor is helically shaped and is provided in such a manner that proper electrical contact between the inner surface and the outer surface of the catheter is established.

6.      A catheter according to one or more of the preceding claims, characterized in that the conductor is strip-shaped and is provided in longitudinal direction of the catheter in such a manner that an electrical connection is established between the contents of the catheter and the medium surrounding the catheter.

7. A catheter according to one or more of the preceding claims, characterized in that a plurality of electrical conductors are provided in the longitudinal direction.

8. A catheter according to one or more of the preceding claims, characterized in that one or more electrical conductors are provided throughout the entire length of the catheter or a portion thereof in such a manner that the conductors alternately establish electrical contact between the contents of the catheter and the medium surrounding the catheter throughout the entire length of the catheter or the portion thereof.

9. A catheter according to one or more of the preceding claims, characterized in that the wall of the catheter is provided with mechanical reinforcements in order to compensate for weakenings possibly resulting from applying the invention.

10. A catheter according to one or more of the preceding claims, characterized in that the wall of the catheter has poor heat conductivity.

11. A catheter according to one or more of the preceding claims, characterized in that an electrical conductor insulated relative to its surroundings is provided in or at the wall of the catheter in the longitudinal direction thereof.

12. A catheter according to one or more of the preceding claims, characterized in that the catheter has been rendered radiopaque.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | US-A-3 659 588 (A.R. KAHN et al.) <br> * Abstract; column 1, lines 4-15; column 2, lines 5-37; column 3, lines 41-60; column 6, lines 11-30; figures 1b,4 * | 1-3,8 | A 61 B 5/04 <br> A 61 M 25/00 |
| | --- | | |
| X | US-A-4 027 659 (E.L. SLINGLUFF) <br><br> * Abstract; column 1, line 60 - column 2, line 1; column 2, line 62 - column 3, line 16; column 3, line 46 - column 4, line 22; figures 1,2 * | 1,2,8 9,12 | |
| | --- | | |
| X | GB-A-1 427 134 (NATIONAL RESEARCH DEVELOPMENT CORP.) <br> * Page 1, lines 26-54; page 1, line 78 - page 2, line 32 * | 1-5,8 | |
| | | | **TECHNICAL FIELDS SEARCHED (Int. Cl. ³)** |
| | --- | | |
| A | US-A-3 070 132 (D.S. SHERIDAN) <br><br> * Column 2, lines 17-32; column 3, lines 3-12, 56-60; column 3, line 71 - column 4, line 24; figures 1-5 * | 1,4-8 11 | A 61 B <br> A 61 M |
| | --- | | |
| A | US-A-4 385 635 (O.F. RUIZ) <br> * Abstract; column 2, lines 42-58; column 3, lines 10-21; column 3, line 32 - column 4, line 4; figures 1,2 * | 9 | |
| | ---     -/- | | |

The present search report has been drawn up for all claims

| Place of search | Date of completion of the search | Examiner |
|---|---|---|
| THE HAGUE | 14-05-1984 | RIEB K.D. |

| | DOCUMENTS CONSIDERED TO BE RELEVANT | | Page 2 |

| Category | Citation of document with indication, where appropriate, of relevant passages | Relevant to claim | CLASSIFICATION OF THE APPLICATION (Int. Cl. ³) |
|---|---|---|---|
| A | FR-A-2 272 690 (H. GRAUSZ et al.)<br>* Page 3, lines 28-35; page 5, lines 12-14; figure 2 * | 11 | |
| E | NL-A-8 201 165 (W. SPAA)<br>* Whole document * | 1-12 | |

**TECHNICAL FIELDS SEARCHED (Int. Cl. ³)**

The present search report has been drawn up for all claims

| Place of search<br>THE HAGUE | Date of completion of the search<br>14-05-1984 | Examiner<br>RIEB K.D. |
|---|---|---|

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503 03.82